(19) Europäisches Patentamt
European Patent Office
Office européen des brevets

(11) Publication number: **0 032 618**
**B1**

(12) **EUROPEAN PATENT SPECIFICATION**

(45) Date of publication of patent specification: **15.06.83**

(21) Application number: **80304513.7**

(22) Date of filing: **15.12.80**

(51) Int. Cl.³: **B 01 J 23/31**,
B 01 J 23/88, B 01 J 23/36,
B 01 J 23/54,
C 07 C 120/14

(54) **Oxidation and ammoxidation catalysts and their uses.**

(30) Priority: **17.12.79 US 104498**

(43) Date of publication of application:
**29.07.81 Bulletin 81/30**

(45) Publication of the grant of the patent:
**15.06.83 Bulletin 83/24**

(84) Designated Contracting States:
**DE FR GB IT**

(56) References cited:
**EP - A - 0 000 835**
**EP - A - 0 005 067**
**GB - A - 1 314 578**
**US - A - 3 629 317**
**US - A - 4 036 901**
**US - A - 4 093 558**

(73) Proprietor: **MONSANTO COMPANY**
**Patent Department 800 North Lindbergh Boulevard**
**St. Louis, Missouri 63166 (US)**

(72) Inventor: **Ebner, Jerry Rudolph**
**233 Bentwood**
**St. Charles Missouri 63301 (US)**

(74) Representative: **McLean, Peter et al,**
**Monsanto Europe S.A. Patent Department Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 032 618 B1**

Courier Press, Leamington Spa, England.

# Oxidation and ammoxidation catalysts and their uses

## Background of the invention

This invention relates to oxidation and/or ammoxidation catalysts containing the elements bismuth, molybdenum, antimony, an additional element, oxygen, and to a method of preparing such catalysts. In another aspect, this invention relates to a process employing such catalysts.

It is well known that olefins can be oxidized to oxygenated hydrocarbons such as unsaturated aldehydes and acids, for example, acrolein and methacrolein, and acrylic and methacrylic acid. It is also well known that olefins can be ammoxidized to unsaturated nitriles such as acrylonitrile and methacrylonitrile. The value of such oxygenated hydrocarbons and unsaturated nitriles is generally well recognized with acrylonitrile being among the most valuable monomers available to the polymer industry for producing useful polymeric products.

Various catalytic processes are known for the oxidation and/or ammoxidation of olefins. Such processes commonly react an olefin or an olefin-ammonia mixture with oxygen in the vapor phase in the presence of a catalyst. For the production of acrolein and acrylonitrile, propylene is the generally used olefin reactant and for the production of methacrolein and methacrylonitrile, isobutylene is the generally used olefin reactant.

Many catalysts are disclosed as suitable in the foregoing reactions. One such catalyst is described in U.S. Patent 3,681,421. This catalyst employs oxides of antimony, vanadium, and at least one additional polyvalent metal in the proportion of 1 gram atom of antimony, 0.12—0.5 gram atoms of vanadium, and 0.25—0.5 gram atoms of the additional polyvalent metal. Under the conditions of the examples of this patent, a yield of 56% of acrylonitrile was obtained using propylene, ammonia, air and steam as reactants.

More recently, U.S. Patent 4,093,558 discloses a catalyst composed of combined oxides of antimony, molybdenum, one of the group of iron and vanadium, and optionally one or more of a number of elements including bismuth. This catalyst is particularly useful for preparing maleic anhydride from butane by oxidative dehydrogenation.

Preparation of ammoxidation catalysts by preforming bismuth molybdate and then mixing the preformed bismuth molybdate with other elements is disclosed in U.S. Patent 4,040,978.

In European Patent Application A—0 005 067 there is disclosed in Example 5 an oxidation catalyst containing Bi, Mo, Sb and M (namely Fe, Co, Ni) and prepared by forming a bismuth molybdate component separately prior to forming the final aqueous mixture which is then dried and calcined at 550°C. This catalyst gives higher yields and selectivity of conversion of propylene, ammonia and air to acrylonitrile than do prior art catalysts.

In U.S. Patent 3,629,317 the catalyst used for the manufacture of acrylonitrile is also prepared by forming a mixture containing a bismuth molybdate component, the mixture being then calcined at a temperature between 300—700°C. For the catalysts described in Examples 5 and 6, the theoretical and empirical catalyst formula calculated from the catalyst composition given on Table I, Examples 5 and 6, could be expressed as follows:

$$Bi\,Mo_{0.5-0.6}Sn_{0.4}Sb_{0.6-0.7}O_x—$$

U.S. Patent 4,036,901 discloses in Example 18 an oxidation catalyst having the empirical formula $BiMo_{2.4}\,Fe\,Sb_{0.6}$ which is useful for oxidation of vinylcyclohexene to produce styrene.

It is well known that the economics of acrylonitrile manufacture dictate increasingly higher yields and selectivity of conversion of the reactants to acrylonitrile in order to minimize the difficulties attending purification of the product and handling of large recycle streams. Moreover, it is known that prior art catalysts frequently produce relatively large quantities of undesired oxygen-containing by-products such as $CO_2$, acrolein and/or acrylic acid which must be removed in purification of the acrylonitrile.

## Summary of the invention

It is an object of this invention to provide a novel catalyst composition useful in the preparation of unsaturated aliphatic nitriles by ammoxidation of aliphatic olefins.

Yet another object is to provide a catalyst which is useful for oxidation of aliphatic olefins to the corresponding unsaturated aldehyde.

A more specific object of this invention is to provide a catalyst which gives surprisingly higher yields and selectivity of conversion of propylene, ammonia and air to acrylonitrile than do prior art catalysts.

It is a further object to provide a catalyst which minimizes the production of oxygenated byproducts of acrylonitrile, such as $CO_2$, acrolein, acrylic acid and the like.

Still another object is to provide a catalyst which exhibits substantially its full activity immediately upon startup of an ammoxidation process, i.e., which requires no break-in period under ammoxidation conditions in order to exhibit its full efficiency in terms of activity and selectivity.

A further object of this invention is to provide a process for manufacture of such a catalyst.

In another aspect, it is an object of this invention to provide an ammoxidation process which employs such a catalyst.

To achieve these and other objects which will become apparent, a catalyst is provided having the empirical formula of $BiMo_aM_bSb_cO_x$ wherein a is from 0.5 to 2.0, b is from 0.05 to 1.0, c is from 0.1 to 1.5, and x is chosen to satisfy the valence requirements of the other elements present, and wherein M is selected from Mn, Mg, Ag, Cr, Pb, Fe, Sn, Zn, Ce, Co, Ni, In, Ti, Zr, Tl and U. According to the present invention, such a catalyst is prepared by forming a bismuth molybdate, forming a metal antimonate, mixing these two component compounds and calcining the resultant mixture at a temperature of from 500 to 850°C.

As used herein, the term "bismuth molybdate" means a chemical compound of bismuth, molybdenum and oxygen. Such compounds may be formed by the interaction of bismuth compounds in which bismuth acts as a cation with salts of molybdic acids such as ammonium molybdate, for example. The "bismuth molybdate" component of catalysts according to this invention may contain oxides of bismuth and/or molybdenum, in addition to bismuth molybdate compounds.

Likewise, as used herein, the term "metal antimonate" is employed to designate complex mixtures of the selected metal, antimony and oxygen. Compounds which may be formed by the interaction of antimony compounds and the selected metal compounds are intended to be included within the term "metal antimonate" as used herein. The "metal antimonate" component of these catalysts may contain free oxides of antimony and/or the selected metal.

The metal antimonate component may be a compound which is formed under conditions of calcination in air at elevated temperatures; however, as used herein, the term metal antimonate also includes precursors of true metal antimonate compounds. Such precursors are preferably formed by preparing an aqueous slurry of a metal compound and an antimony compound. The slurry is boiled until the contained solids have had an opportunity to react to form a metal antimonate precursor. The metal antimonate precursor may, if necessary, be calcined to form a true metal antimonate compound as will be more fully explained hereinafter.

The bismuth molybdate component of catalysts according to this invention is preferably prepared by forming an aqueous solution of a bismuth salt, forming an aqueous solution of a molybdenum-containing salt, oxide or hydroxide, combining these two solutions, and recovering the precipitated bismuth molybdate by filtration or decantation. Suitable bismuth compounds include, but are not limited to bismuth metal when used as a solution with nitric acid, bismuth oxide, bismuth trinitrate, pentahydrate and bismuth halides when hydrolyzed. Similarly, suitable molybdenum compounds include molybdenum trioxide, molybdic acid, ammonium molybdates, such as ammonium dimolybdate and ammonium heptamolybdate and molybdenum halides.

The bismuth molybdate component of catalysts according to this invention is preferably prepared in the absence of the metal antimonate component. However, the bismuth molybdate component may, if desired, be prepared in the presence of preformed metal antimonate.

It is known that bismuth molybdate may exist in alpha, beta and gamma forms or phases. An interesting and informative source of information on these forms of bismuth molybdate is found in *Journal of Solid State Chemistry*, Vol. *13*, pp. 228—297 (1975), authored by Tu Chen and G. S. Smith. The alpha bismuth molybdate has the molecular formula $Bi_2(MoO_4)_3$, while the beta form is $Bi_2Mo_2O_9$ and the gamma form has the formula $Bi_2MoO_6$. Particularly preferred catalysts according to this invention employ predominantly the beta bismuth molybdate phase with lesser amounts of the alpha phase present.

The metal antimonate component of these catalysts is preferably formed by addition of a metal-containing compound to an aqueous slurry of an antimony-containing compound and drying the resultant slurry. In a preferred preparation, the metal and antimony compounds are the oxides. The slurry is boiled until the contained solids exhibit a color change indicating the interaction or partial reaction of the two metal compounds to form a precursor of the metal antimonate. In a particularly preferred embodiment of this invention, the metal antimonate precursor is dried and calcined for one-half to 24 hours at a temperature of 500 to 800°C, preferably 550 to 750°C to form a stable metal antimonate compound.

Although it is preferred to use the oxides of the metal and antimony as described above in preparing the antimonate component of the catalyst, other antimony and metal-containing compounds may be employed. Exemplary of such antimony compounds are antimony trioxide, antimony tetroxide, antimony pentoxide and antimonic acid. Compounds which form an oxide of antimony after chemical reaction or calcination are also suitable, for example, metallic antimony, antimony hydroxides and antimony halides, such as antimony trichloride, tribromide and antimony pentachloride. Suitable metal compounds include the oxides, nitrates, acetates, oxalates and halides.

Following preparation of the bismuth molybdate and metal antimonate components to the catalyst, these components are preferably combined in the desired proportions in an aqueous slurry and well mixed. At this point a suitable catalyst support may be added, if desired, as described below.

The slurry, after intimate mixing, is then heated to remove the bulk of the aqueous phase. The concentrated slurry contains a certain amount of water and it is desirable to remove this water by some

form of drying process to form a dry catalyst precursor. This can take the form of a simple oven-drying process in which the water containing solid phase is subjected to a temperature that is sufficiently high to vaporize the water and completely dry the solid phase.

An alternate drying process which may be employed is the so-called spray-drying process in which water-containing solid phase particles are sprayed into contact with hot gas (usually air) so as to vaporise the water. The drying is controlled by the temperature of the gas and the distance the particles travel in contact with the gas. It is generally undesirable to adjust these parameters to achieve too rapid drying as this results in a tendency to form dried skins on the partially dried particles of the solid phase which are subsequently ruptured as water occluded within the particles vaporized and attempts to escape. By the same token, it is desirable to provide the catalyst in a form having as little occluded water as possible. Therefore, where a fluidized bed reactor is to be used and microspheroidal particles are desired, it is advisable to choose the conditions of spray-drying with a view of achieving substantially complete drying without particle rupture.

Following the drying operation, the catalyst precursor is calcined to form the catalyst. The calcination process is usually conducted in air at essentially atmospheric pressure and at a temperature of 500° to 850°C, such as from about 525°C to about 650°C. The time to complete the calcination can be anything up to 10 hours, but for most purposes, the calcination need take only from about 1 to 2 hours.

In some applications, it may be advantageous to include in the catalyst a support material which functions by providing a large surface area for the catalyst and by creating a harder and more durable catalyst for use in the highly abrasive environment of a fluidized bed reactor. This support material can be any of those commonly proposed for such use, such as, for example, silica, zirconia, alumina, titania, antimony pentoxide sol or other oxide substrates. From the point of view of availability, cost and performance, silica is usually a satisfactory support material and is preferably in the form of silica sol for easy dispersion.

In a less preferred embodiment of this invention, the metal antimonate component and bismuth molybdate component of the catalyst may be mixed in dry form, optionally, with a support material and then calcined in air.

The proportions in which the components of the supported catalyst are present can vary widely, but it is usually preferred that the support provides from 10 to 90% and more preferably about 35 to 60% by weight of the total combined weight of the catalyst and the support. To incorporate a support into the catalyst, the support material is preferably added to the slurry containing the active components discussed above.

As has been stated, catalysts according to this invention are those having the empirical formula $BiMo_aM_bSb_cO_x$, where a is from 0.5—2, b is from 0.05—1, c is from 0.1—1.5 and x is taken to satisfy the valence requirements of the other elements present. In more preferred embodiments of such catalysts, a is from 0.7—1.5, b is from 0.08—1 and c is from 0.1—1.2.

The catalyst preparation of the invention yields a catalyst that is particularly useful in the production of acrylonitrile from propylene and in which follows specific reference is made to that process although it should be understood that the described catalyst is also useful for ammoxidation of other olefins and for oxidation of aliphatic olefins to aldehydes and acids.

In the most frequently used ammoxidation processes, a mixture of olefin, ammonia and oxygen (or air) is fed into a reactor and through a bed of catalyst particles. The reaction temperature is usually in the range of 400°C to 550°C and preferably 450°C to 525°C, and the pressure is 1 to 6 atmospheres (101 to 608 kPa). The ammonia and olefin are required stoichimetrically in equimolar amounts, but it is usually necessary to operate with a molar ratio of ammonia to olefin in excess of 1 to reduce the incidence of side reactions. Likewise, the stoichiometric oxygen requirement is 1.5 times the molar amount of olefin. The feed mixture is commonly introduced into the catalyst bed at a W/F (defined as the weight of the catalyst in grams divided by the flow of reactant stream in ml/sec. at standard temperature and pressure) in the range of 2 to 15, preferably from about 4 to about 10.

The ammoxidation reaction is exothermic and for convenience in heat distribution and removal, the catalyst bed is desirably fluidized, however, fixed catalyst beds may be employed with alternative heat removal means such as cooling coils within the bed.

The catalyst prepared by the process of the present invention is particularly well adapted for use in such a process and in which follows its effectiveness and advantages over prior art catalysts are demonstrated in the context of that process.

Specific embodiments

As has been stated above, the catalyst of the invention has the empirical formula $BiMo_aM_bSb_cO_x$, wherein a is from 0.5 to 2.0, b is from 0.05 to 1.0 and c is from 0.1 to 1.5 and x is a number taken to satisfy the valence requirements of the other elements present in the catalyst and wherein M is selected from Mn, Mg, Ag, Cr, Pb, Fe, Sn, Zn, Ce, Co, Ni, In, Ti, Zr, U and Tl, optionally dispersed on a finely divided support which represents from 10 to 90% of the supported catalyst weight. In particularly preferred embodiments, M is selected from Mn, Sn, Fe, Zn, Ag, Cr and Mg. In the examples that are

presented below, specific compositions within this range were prepared and employed as catalysts in the ammoxidation of propylene to produce acrylonitrile.

As used in the following examples, the following terms are defined in the following manner:

1. "W/F" is defined as the weight of the catalyst in grams divided by the flow rate of reactant stream in ml/sec. measured at S.T.P.

2. "Propylene ($C_3H_6$) conversion" is defined as:

$$\frac{\text{Mols } C_3H_6 \text{ in feed—mols } C_3H_6 \text{ in effluent}}{\text{Mols } C_3H_6 \text{ in feed}} \times 100\%$$

3. "Acrylonitrile (AN) selectivity" is defined as:

$$\frac{\text{Mols AN in effluent}}{\text{Mols } C_3H_6 \text{ converted}} \times 100\%$$

4. "Acrylonitrile (AN) yield" is defined as:

$$\frac{\text{Mols AN formed}}{\text{Mols } C_3H_6 \text{ feed}} \times 100\%$$

In the following examples, unless otherwise noted, the catalysts of the examples were evaluated to determine acrylonitrile selectivity and yield and propylene conversion in a fluidized bed reaction vessel having an inside diameter of about 13 millimeters. Approximately 25 grams of catalyst was used in each case. A reactant mixture of 17—17.8 volume % $O_2$, 7.6—8.3 volume % propylene ($C_3H_6$), 8—9 volume % $NH_3$, and the balance helium was passed upward through the catalyst bed at a rate sufficient to give the value of W/F shown in the experimental results for each example. The temperatures shown in the examples are expressed in degrees Celsius and in each instance the pressure at which the reaction was carried out was 205 kPa.

In each of the following examples the bismuth molybdate and the final catalyst were prepared according to the following generalized procedures. The metal antimonate component of each catalyst was prepared by a standardized procedure differing from example to example only in the specific metal compound employed as the metal source and in the calcination conditions for the antimonate component.

Preparation of bismuth molybdate component

100 grams of molybdenum oxide ($MoO_3$) were dissolved in a mixture of 1000 ml of water and 150 ml of concentrated (58%) ammonium hydroxide, added to a solution of 340 grams of bismuth nitrate pentahydrate in about 200 ml of water and 200 ml of concentrated nitric acid with stirring and the pH of the resulting mixture was adjusted to about 3.65 using 29% ammonium hydroxide and diluted to a total volume of 3500 ml with water. The slurry was then heated and stirred for appoximately 3 hours. The resulting precipitate was filtered and washed thoroughly with distilled water.

Preparation of antimonate component

A slurry was formed of $Sb_2O_3$ and distilled water in the approximate ratio of 1 gram $Sb_2O_3$ to 1 ml water. To this slurry was added the metal compound shown in the following Table (Column 2) which had previously been finely ground in a mortar and pestle. The resultant slurry was heated at 50—80°C with stirring until almost dry and then heated to about 130°C for several hours to dry it thoroughly. Finally, the dried metal antimonate precursor was calcined in air for two hours at the temperature shown in the following Table (Column 3). The only exception to the two-hour calcination was in Example 2 in which the manganese antimonate precursor was calcined for 6 hours.

Preparation of catalyst

A slurry of 90 grams of a silica sol containing 40% silica was adjusted to a pH of 3.5 with 10% nitric acid and added with vigorous stirring to the appropriate amount of bismuth molybdate component prepared as above. The antimonate component was then added to the prescribed amount. The catalyst mixture was stirred and heated to obtain a homogeneous dispersion of ingredients. The mixture was thoroughly tray dried and then calcined at 550°C for one hour in air to obtain the final catalyst having catalytic elements in the atomic proportions reported in the examples.

TABLE

| | Antimonate component | | Finished catalyst | | Catalyst performance | | | | |
|---|---|---|---|---|---|---|---|---|---|
| Example | Starting metal compound | Calcination temp. (°C) | Empirical formula | Silica (%) | W/F | Temp. (°C) | %AN yield | %AN selectivity | %Propylene conversion |
| 1 | $MnO_2$ | 700 | $BiMo_1Mn_{0.5}Sb_1O_x$ | 50 | 5.4 | 465 | 69 | 74 | 93 |
| 2 | $Mn(C_2H_3O_2)\cdot 4H_2O$ | 6 hrs @ 700 | $BiMo_1Mn_{0.375}Sb_{0.75}O_x$ | 42 | 4.8 | 472 | 72 | 75 | 95 |
| 3 | $CeO_2$ | 650 | $BiMo_1Ce_{.25}Sb_1O_x$ | 50 | 4.0 | 455 | 67 | 71 | 95 |
| 4 | $CeO_2$ | 650 | $BiMo_1Ce_{.4}Sb_1O_x$ | 50 | 5.0 | 470 | 69 | 72 | 96 |
| 5 | $Ce(NO_3)_4\cdot(NH_4NO_3)_2$ | 6 hrs @ 750 | $BiMo_1Ce_{.48}Sb_1O_x$ | 45 | 5.2 | 460 | 65 | 71 | 92 |
| 6 | $PbO_2$ | 700 | $BiMo_1Pb_{.5}Sb_1O_x$ | 50 | 7.0 | 455 | 54 | 67 | 81 |
| 7 | $PbO_2$ | 700 | $BiMo_1Pb_{.25}Sb_1O_x$ | 50 | 6.5 | 455 | 64 | 73 | 88 |
| 8 | $SnO$ | 800 | $BiMo_1Sn_{.33}Sb_1O_x$ | 45 | 3.2 | 470 | 70 | 75 | 94 |
| 9 | $Fe(NO_3)_3\cdot 9H_2O$ | 750 | $BiMo_1Fe_{.33}Sb_1O_x$ | 45 | 4.2 | 470 | 71 | 76 | 93 |
| 10 | $ZnCO_3$ | 750 | $BiMo_1Zn_{.25}Sb_1O_x$ | 45 | 3.2 | 475 | 71 | 74 | 95 |
| 11 | $Ag_2CO_3$ | 800 | $BiMo_1Ag_{.05}Sb_{.5}O_x$ | 45 | 3.9 | 475 | 73 | 77 | 95 |
| 12 | $Ag_2CO_3$ | 800 | $BiMo_1Ag_{0.1}Sb_1O_x$ | 45 | 5.0 | 465 | 73 | 76 | 95 |
| 13 | $Ag_2CO_3$ | 600 | $BiMo_1Ag_{0.25}Sb_1O_x$ | 50 | 5.4 | 465 | 66 | 73 | 91 |
| 14 | $CrO_3$ | 700 | $BiMo_1Cr_{.25}Sb_{.5}O_x$ | 45 | 5.1 | 457 | 73 | 77 | 95 |
| 15 | $CrO_3$ | 700 | $BiMo_1Cr_{.5}Sb_1O_x$ | 45 | 6.5 | 450 | 70 | 75 | 94 |
| 16 | $CrO_3$ | 600 | $BiMo_1Cr_{.25}Sb_1O_x$ | 50 | 4.0 | 465 | 71 | 75 | 95 |
| 17 | $MgCO_3$ | 650 | $BiMo_1Mg_{.25}Sb_{.5}O_x$ | 45 | 2.9 | 480 | 72 | 76 | 95 |
| 18 | $MgCO_3$ | 650 | $BiMo_1Mg_{.5}Sb_1O_x$ | 45 | 3.8 | 465 | 69 | 74 | 94 |
| 19 | $MgCO_3$ | 650 | $BiMo_1Mg_{.25}Sb_1O_x$ | 50 | 5.4 | 453 | 67 | 72 | 93 |
| 20 | $Co_2O_3$ | 750 | $BiMo_1Co_{.5}Sb_1O_x$ | 50 | 5.0 | 455 | 66 | 73 | 91 |
| 21 | $CoCO_3$ | 700 | $BiMo_1Co_{.25}Sb_1O_x$ | 50 | 4.0 | 455 | 63 | 72 | 88 |
| 22 | $In_2O_3$ | 650 | $BiMo_1In_{.4}Sb_1O_x$ | 50 | 4.4 | 455 | 66 | 71 | 94 |
| 23 | $NiCO_3$ | 700 | $BiMo_1Ni_{.25}Sb_1O_x$ | 50 | 5.1 | 465 | 70 | 74 | 95 |
| 24 | $NiCO_3$ | 650 | $BiMo_1Ni_{.5}Sb_1O_x$ | 50 | 5.1 | 465 | 68 | 73 | 94 |

## Claims

1. A catalyst for oxidation and ammoxidation of hydrocarbons characterized by catalytic elements having the empirical formula

$$BiMo_aM_bSb_cO_x$$

wherein a is from 0.5 to 2, b is from 0.05 to 1, c is from 0.1 to 1.5 and x is taken to satisfy the valence requirements of the other elements present and wherein M is a metal selected from Mn, Mg, Cr, Pb, Fe, Sn, Zn, Ag, Ti, Ce, Co, Ni, In, Zr, Tl, U and mixtures thereof, said catalyst being prepared by forming a mixture containing a selected "metal antimonate" component, a bismuth molybdate component and, optionally, a support material and calcining at a temperature of from 500 to 850°C.

2. A catalyst according to Claim 1 characterized in that M is selected from Mn, Sn, Fe, Zn, Ag, Cr and Mg.

3. A catalyst according to Claim 1 characterized in that a is from 0.7 to 1.5, b is from 0.08 to 1 and c is from 0.1 to 1.2.

4. A catalyst according to Claim 2 characterized in that said antimonate component is calcined prior to forming said mixture.

5. A catalyst according to Claim 4 characterized in that said bismuth molybdate component is separately formed prior to forming said mixture.

6. A catalyst according to Claim 4 characterized in that said metal antimonate component is preformed and said bismuth molybdate component is formed in the presence of said preformed antimonate component.

7. A catalyst according to Claim 5 characterized in that said mixture is in the form of an aqueous slurry.

8. A catalyst according to Claim 7 characterized in that a support material representing from 10 to 90% of the total weight of the supported catalyst is added to said slurry.

9. A catalyst according to Claim 4 characterized in that said mixture contains an excess of an antimony compound over that stoicheometrically required to combine with the selected metal in said metal antimonate component.

10. A process for the production of acrylonitrile which comprises reacting at an elevated temperature in the vapor phase propylene, ammonia and molecular oxygen over a catalyst characterized by catalytic elements having the empirical formula

$$BiMo_aM_bSb_cO_x$$

wherein a is from 0.5 to 2, b is from 0.05 to 1, c is from 0.1 to 1.5 and x is taken to satisfy the valence requirements of the other elements present and wherein M is a metal selected from Mn, Mg, Cr, Pb, Fe, Sn, Zn, Ag, Ti, Ce, Co, Ni, In, Zr, Tl, U and mixtures thereof, said catalyst being prepared by forming a mixture containing a selected metal antimonate component, a bismuth molybdate component, and, optionally, a support material and calcining at a temperature of from 500 to 850°C.

11. A process according to Claim 10 characterized in that M is selected from Mn, Sn, Fe, Zn, Ag, Cr and Mg.

12. A process according to Claim 10 characterized in that a is from 0.7 to 1.5, b is from 0.08 to 1 and c is from 0.1 to 1.2.

13. A process according to Claim 12 characterized in that said mixture is an aqueous slurry.

14. A process according to Claim 13 characterized in that said antimonate component is calcined prior to forming said slurry.

15. A process according to Claim 14 characterized in that said metal antimonate component and said bismuth molybdate component are separately formed prior to forming said slurry.

16. A process according to Claim 14 characterized in that said metal antimonate component is preformed and said bismuth molybdate component is formed in the presence of said preformed antimonate component.

17. A process according to Claim 15 characterized in that a support material representing from 10 to 90% of the total weight of the supported catalyst is added to said slurry.

18. A process according to Claim 14 characterized in that said slurry contains an excess of an antimony compound over that stoicheometrically required to combine with the selected metal in said metal antimonate component.

19. A process according to Claim 17 characterized in that said process is conducted at a pressure of 101 to 608 kPa and a temperature of 400 to 550°C.

20. A process according to Claim 19 characterized in that said temperature is 450 to 525°C.

21. A process according to Claim 19 characterized in that the flow rate W/F is in the range of 2 to 15 gm sec/ml.

**Patentansprüche**

1. Katalysator zur Oxidation und Ammoxidation von Kohlenwasserstoffen, gekennzeichnet durch katalytische Elemente der empirischen Formel

$$BiMo_aM_bSb_cO_x$$

worin a einen Wert von 0,5 bis 2, b einen Wert von 0,05 bis 1 und c einen Wert von 0,1 bis 1,5 bedeutet und x ausgewählt ist, um die Wertigkeits-Erfordernisse der anderen vorhandenen Elemente zu erfüllen, und worin M ein Metall ist, welches unter Mn, Mg, Cr, Pb, Fe, Sn, Zn, Ag, Ti, Ce, Co, Ni, In, Zr, Tl, U und Gemischen davon ausgewählt ist, wobei der Katalysator durch Bildung eines Gemisches, welches eine ausgewählte Metallantimonat-Komponente, eine Wismutmolybdat-Komponente und gegebenenfalls ein Trägermaterial enthält, und Calcinieren bei einer Temperatur von 500 bis 850°C hergestellt wird.

2. Katalysator nach Anspruch 1, dadurch gekennzeichnet, daß M unter Mn, Sn, Fe, Zn, Ag, Cr und Mg ausgewählt ist.

3. Katalysator nach Anspruch 1, dadurch gekennzeichnet, daß a einen Wert von 0,7 bis 1,5, b einen Wert von 0,08 bis 1 und c einen Wert von 0,1 bis 1,2 aufweist.

4. Katalysator nach Anspruch 2, dadurch gekennzeichnet, daß die Antimonat-Komponente vor der Bildung des Gemisches calciniert wird.

5. Katalysator nach Anspruch 4, dadurch gekennzeichnet, daß die Wismutmolybdat-Komponente vor der Bildung des Gemisches getrennt gebildet wird.

6. Katalysator nach Anspruch 4, dadurch gekennzeichnet, daß die Metallantimonat-Komponente vorgeformt wird und daß die Wismutmolybdat-Komponente in Gegenwart der vorgeformten Antimonat-Komponente gebildet wird.

7. Katalysator nach Anspruch 5, dadurch gekennzeichnet, daß das Gemisch in Form einer wäßrigen Aufschlämmung vorliegt.

8. Katalysator nach Anspruch 7, dadurch gekennzeichnet, daß ein Trägermaterial, welches 10 bis 90% des Gesamtgewichts des Trägerkatalysators darstellt, der Aufschlämmung zugesetzt wird.

9. Katalysator nach Anspruch 4, dadurch gekennzeichnet, daß das Gemisch einen Überschuß an einer Antimon-Verbindung über die stöchiometrische Menge hinaus, die erforderlich ist, um mit dem ausgewählten Metall in der Metallantimonat-Komponente zu kombinieren, enthält.

10. Verfahren zur Herstellung von Acrylnitril, wobei bei erhöhter Temperatur in der Dampfphase Propylen, Ammoniak und molekularer Sauerstoff über einem Katalysator umgesetzt werden, der gekennzeichnet ist durch katalytische Elemente der empirischen Formel

$$BiMo_aM_bSb_cO_x$$

worin a einen Wert von 0,5 bis 2, b einem Wert von 0,05 bis 1 und c einen Wert von 0,1 bis 1,5 bedeutet und x ausgewählt ist, um die Wertigkeits-Erfordernisse der anderen vorhandenen Elemente zu erfüllen, und worin M ein Metall ist, welches unter Mn, Mg, Cr, Pb, Fe, Sn, Zn, Ag, Ti, Ce, Co, Ni, In, Zr, Tl, U und Gemischen davon ausgewählt ist, wobei der Katalysator durch Bildung eines Gemisches, welches eine ausgewählte Metallantimonat-Komponente, eine Wismutmolybdat-Komponente und gegebenenfalls ein Trägermaterial enthält, und Calcinieren bei einer Temperatur von 500 bis 850°C hergestellt wird.

11. Verfahren nach Anspruch 10, dadurch gekennzeichnet, daß M unter Mn, Sn, Fe, Zn, Ag, Cr und Mg ausgewählt ist.

12. Verfahren nach Anspruch 10, dadurch gekennzeichnet, daß a einen Wert von 0,7 bis 1,5, b einen Wert von 0,08 bis 1 und c einen Wert von 0,1 bis 1,2 besitzt.

13. Verfahren nach Anspruch 12, dadurch gekennzeichnet, daß das Gemisch eine wäßrige Aufschlämmung ist.

14. Verfahren nach Anspruch 13, dadurch gekennzeichnet, daß die Antimonat-Komponente vor der Bildung der Aufschlämmung calciniert wird.

15. Verfahren nach Anspruch 14, dadurch gekennzeichnet, daß die Metallantimonat-Komponente und die Wismutmolybdat-Komponente vor der Bildung der Aufschlämmung getrennt gebildet werden.

16. Verfahren nach Anspruch 14, dadurch gekennzeichnet, daß die Metallantimonat-Komponente vorgeformt wird und daß die Wismutmolybdat-Komponente in Gegenwart der vorgeformten Antimonat-Komponente gebildet wird.

17. Verfahren nach Anspruch 15, dadurch gekennzeichnet, daß ein Trägermaterial, welches von 10 bis 90% des gesamtgewichts des Trägerkatalysators darstellt, der Aufschlämmung zugesetzt wird.

18. Verfahren nach Anspruch 14, dadurch gekennzeichnet, daß die Aufschlämmung einen Überschuß einer Antimon-Verbindung über die stöchiometrisch benötigte Menge hinaus, um mit dem ausgewählten Metall in der Metallantimonat-Komponente zu kombinieren, enthält.

19. Verfahren nach Anspruch 17, dadurch gekennzeichnet, daß das Verfahren unter einem Druck von 101 bis 608 kPa und einer Temperatur von 400 bis 550°C durchgeführt wird.

20. Verfahren nach Anspruch 19, dadurch gekennzeichnet, daß die Temperatur 450 bis 525°C beträgt.

21. Verfahren nach Anspruch 19, dadurch gekennzeichnet, daß die Fließgeschwindigkeit W/F im Bereich von 2 bis 15 g sec/ml liegt.

**Revendications**

1. Catalyseur pour l'oxydation et l'ammoxydation d'hydrocarbures, caractérisé par des éléments catalytiques ayant la formule:

$$BiMo_aM_bSb_cO_x$$

où a est de 0,5 à 2, b est de 0,05 à 1, c est de 0,1 à 1,5 et x est pris pour satisfaire aux exigences de valence des autres éléments présents et où M est un métal choisi parmi Mn, Mg, Cr, Pb, Fe, Sn, Zn, Ag, Ti, Ce, Co, Ni, In, Zr, Tl U et leurs mélanges, ce catalyseur étant préparé en formant un mélange contenant un composant choisi d'"antimoniate métallique", un composant de molybdate de bismuth et, de manière facultative, une matière de support et en calcinant à une température de 500 à 850°C.

2. Catalyseur selon la revendication 1, caractérisé en ce que M est choisi parmi Mn, Sn, Fe, Zn, Ag, Cr et Mg.

3. Catalyseur selon la revendication 1, caractérisé en ce que a est de 0,7 à 1,5, b est de 0,08 à 1 et c est de 0,1 à 1,2.

4. Catalyseur selon la revendication 2, caractérisé en ce que le composant d'antimoniate est calciné avant la formation du mélange.

5. Catalyseur selon la revendication 4, caractérisé en ce que le composant de molybdate de bismuth est séparément formé avant formation du mélange.

6. Catalyseur selon la revendication 4, caractérisé en ce que le composant d'antimoniate métallique est préformé et le composant de molybdate de bismuth est formé en présence du composant d'antimoniate préformé.

7. Catalyseur selon la revendication 5, caractérisé en ce que le mélange est sous la forme d'une boue aqueuse.

8. Catalyseur selon la revendication 7, caractérisé en ce qu'une matière de support représentant 10 à 90% du poids total du catalyseur placé sur support est ajoutée à la boue.

9. Catalyseur selon la revendication 4, caractérisé en ce que le mélange contient un excès d'un composé d'antimoine par rapport à celui stoechiométriquement exigé pour la combinaison avec le métal choisi dans le composant d'antimoniate métallique.

10. Procédé pour la production d'acrylonitrile, qui consiste à faire réagir, à une température élevée, en phase vapeur, du propylène, de l'ammoniac et de l'oxygéne moléculaire sur un catalyseur, caractérisé par des éléments catalytiques ayant la formule empirique:

$$BiMo_aM_bSb_cO_x$$

où a est de 0,5 à 2, b est de 0,05 à 1, c est de 0,1 à 1,5 et x est pris pour satisfaire aux exigences de valence des autres éléments présents et où M est un métal choisi parmi Mn, Mg, Cr, Pb, Fe, Sn, Zn, Ag, Ti, Ce, Co, Ni, In, Zr, Tl, U et leurs mélanges, ce catalyseur étant préparé en formant un mélange contenant un composant choisi d'antimoniate métallique, un composant de molybdate de bismuth et, de manière facultative, une matière de support et en calcinant à une température de 500 à 850°C.

11. Procédé selon la revendication 10, caractérisé en ce que M est choisi parmi Mn, Sn, Fe, Zn, Ag, Cr et Mg.

12. Procédé selon la revendication 10, caractérisé en ce que a est de 0,7 à 1,5, b est de 0,08 à 1 et c est de 0,1 à 1,2.

13. Procédé selon la revendication 12, caractérisé en ce que le mélange est une boue aqueuse.

14. Procédé selon le revendication 13, caractérisé en ce que le composant d'antimoniate est calciné avant la formation de cette boue.

15. Procédé selon la revendication 14, caractérisé en ce que le composant d'antimoniate métallique et le composant de molybdate de bismuth sont séparément formés avant la formation de la boue.

16. Procédé selon la revendication 14, caractérisé en ce que le composant d'antimoniate métallique est préformé et le composant de molybdate de bismuth est formé en présence du composant d'antimoniate préformé.

17. Procédé selon la revendication 15, caractérisé en ce que la matière de support représentant 10 à 90% du poids total du catalyseur placé sur support est ajoutée à la boue.

18. Procédé selon la revendication 14, caractérisé en ce que la boue contient un excès d'un

composé d'antimoine par rapport à celui stoechiométriquement exigé pour se combiner avec le métal choisi dans le composant d'antimoniate métallique.

19. Procédé selon la revendication 17, caractérisé en ce que le procédé est conduit sous une pression de 101 à 608 kPa et à une température de 400 à 550°C.

20. Procédé selon la revendication 19, caractérisé en ce que la température est de 450°C à 525°C.

21. Procédé selon la revendication 19, caractérisé en ce que le débit W/F est dans la gamme de 2 à 15 g.s/ml.